# EUROPÄISCHE PATENTSCHRIFT

(11) **EP 2 135 628 B1**
(45) Veröffentlichungstag und Bekanntmachung des Hinweises auf die Patenterteilung: **02.01.2013**
(21) Anmeldenummer: 09160151.8
(22) Anmeldetag: 13.05.2009
(51) Int. Cl.: A61L 31/02, A61L 31/08, A61L 31/14

(54) **Stent mit einer Beschichtung und einem Grundkörper, die ein Lithiumsalz enthalten**
Stent with a coating and a base body containing a lithium salt
Stent doté d'un revêtement et d'un corps de base comprenant du sel de lithium

(30) Priorität: 17.06.2008 DE 102008002471
(43) Veröffentlichungstag der Anmeldung: 23.12.2009
(73) Patentinhaber: Biotronik VI Patent AG, 6341 Baar (CH)
(72) Erfinder: Wittchow, Eric, 90403, Nürnberg (DE)
(74) Vertreter: Lindner-Vogt, Karin L.

(56) Entgegenhaltungen:
- EP-A2- 0 966 979
- EP-A2- 1 795 215
- EP-A2- 1 891 996
- EP-A2- 2 136 853
- DE-A1-102005 018 356

## Beschreibung

### Technisches Gebiet

Die Erfindung betrifft einen Stent mit einem ggf. beschichteten Grundkörper aus einem Implantatwerkstoffsowie die Verwendung von Lithiumsalzen als Beschichtungsmaterial und Bestandteil eines Implantatwerkstoffs für Stents.

### Technologischer Hintergrund und Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen rohrförmigen Grundkörper mit einer filigranen Tragstruktur aus metallischen Streben auf, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper des Stents besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Eine biologische Reaktion auf Implantatwerkstoffe hängt ab von der Konzentration, Einwirkdauer und Art der Zuführung. Häufig führt allein die Gegenwart eines Implantatwerkstoffs zu Entzündungsreaktionen, deren Auslöser mechanische Reize, chemische Stoffe, aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Ein aussichtsreicher Ansatz zur Lösung des Problems liegt in der Verwendung biokorrodierbarer Metalle und deren Legierungen als Implantatwerkstoff, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest <1% bekannt, die sich insbesondere zur Herstellung einer Endoprothese, zum Beispiel in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen.

Bekannt ist ferner, dass sich ein höheres Maß an Biokompatibilität und damit eine Verbesserung der Restenoserate erreichen lässt, wenn Implantatwerkstoffe mit Beschichtungen aus besonders gewebeverträglichen Materialien versehen werden. Diese Materialien sind zumeist organischer oder synthetisch-polymerer Natur und teils natürlichen Ursprungs.

Werden als Implantatwerkstoff oder als Beschichtungsmaterial biokorrodierbare Polymere eingesetzt, so ist zu beachten, dass die häufig sauren Produkte des Abbaus dieser Polymere zu einer inflammatorischen Reaktion des umgebenden Gewebes führen können, d.h. das Material zeigt nur eine mäßige Biokompatibilität. So wurde zum Beispiel nachgewiesen, dass bei biodegradierbaren Poly(orthoestern) weder die Monomere noch die Zwischenprodukte während der Degradation für die Inflammation verantwortlich sind, sondern die in geringen Spuren freiwerdende Essigsäure (Zignani et al., Subconjunctival biocompatibility of a viscous bioerodible poly(ortho ester), J. Biomed. Mater. Res., 1997, 39 p. 277 - 285). Neben der unerwünschten biologischen Antwort auf die sauren Degradationsprodukte beeinflusst der geänderte pH-Wert im Falle eines Implantats aus einer biokorrodierbaren Magnesiumlegierung mit einer Beschichtung aus einem solchen Polymer auch das Abbauverhalten der Legierung: die saure Umgebung wird den Abbau beschleunigen. Dadurch verliert zum Beispiel ein Stent aus einer biokorrodierbaren Magnesiumlegierung schneller seine Stützkraft.

Eine weitere Strategie zur Vermeidung der Restenose sieht vor, die Proliferation durch Medikation zu hemmen. Es sind wirkstoffbeschichtete Stents (auch als DES bezeichnet: drug eluting stent) bekannt, deren Wirkstoffe nachweislich die Proliferation glatter humaner Gefäßmuskelzellen unterbinden; Beispiele umfassen die Wirkstoffe Sirolimus und Paclitaxel. Nachteilig sind die zur Vermeidung einer Spät-Thrombose notwendige Begleitmedikation, die hohen Kosten der bisher zum Zwecke der Restenoseprophylaxe eingesetzten Wirkstoffe sowie deren aufwendige Verarbeitung.

### Zusammenfassung der Erfindung

Der Erfindung liegt die Aufgabe zugrunde, ein oder mehrere der geschilderten Nachteile des Standes der Technik zu mindern oder zu überwinden.

Die Erfindung geht aus von einem Stent mit einem Grundkörper aus einem Implantatwerkstoff. Der erfindungsgemäße Stent zeichnet sich dadurch aus, dass
(i) der Grundkörper eine Beschichtung aufweist, die aus einem Lithiumsalz besteht oder ein Lithiumsalz enthält; und
(ii) der Implantatwerkstoff biokorrodierbar ist und der Grundkörper ein Lithiumsalz enthält und das Lithiumsalz basisch ist.

Es hat sich gezeigt, dass Lithiumsalze einen dilatierenden Einfluss auf Blutgefäße haben. Diese Gefäßdilatation wirkt sich positiv auf den Durchmesser des Gefäßes im Bereich der Läsion aus, die vom dem Stent abgestützt wird (Dehpour AR, Aghadadashi H, Ghafourifar P, Roushanazamir F, Ghahremani MH, Meysamee F, Rassaee N, Koucharian A. Effect of chronic lithium administration on endothelium-dependent relaxation in rat aorta. Clin Exp Pharmacol. 2000 Jan-Feb ;27(1-2) :55-9). Lithiumsalze werden seit langen zur Behandlung psychischer Störungen, zum Beispiel bipolarer Affektstörungen, Manie und Depressionen, eingesetzt. In empirischen Studien konnte ein Zusammenhang der Lithiumgabe und einer Abnahme von Krebserkrankungen nachgewiesen werden (Cohen Y, Chetrit A, Cohen Y, Sirota P, Modan B, Cancer morbidity in psychatric patients: influence oh lithum carbonate treatment. Med Oncol. 1998 Apr;15(1):32-6). Letztere Wirkung beruht dabei sehr wahrscheinlich auf der Beeinflussung des Isonitol-Stoffwechsels durch Hemmung der myo-lsonitol-1-Phosphatase unter der Hemmung der Gykogensynthasekinase-3 (GSK-3) in Nervenzellen (Berridge MJ. Inositol triphosphate and diacylglycerol as second messenger. Biochem J. 1984 Jun1;220(2):345-60., Carney DH, Scott DL, Gordon EA, LaBelle EF. Phosphoinositides in mitogenesis: neomvcin inhibits thrombin-simulated phosphoinositide turnover and initation of cell proliferation. Cell. 1985 Sep;42(2):479-88., Williams R, Ryves WJ, Daltin EC, Eickholt B, Shaltiel G, Agam G, Harwood AJ. A molecular cell biology of lithium. Biochem Soc Trans. 2004 Nov;32(Pt 5):799-802). In Zellkulturversuchen unterdrückt Lithiumchlorid signifikant die Proliferation verschiedener Prostata-Krebszelllinien durch Vermindung der Genexpression in der S-Phase des Zellzyklus (Sun A, Shanmugam I, Song J, Terranove PF, Thrasher JB, Li B. Lithium sippresses cell proliferation by interrupting E2F-DANN interaction and subsequently reducing S-phase gene expression in prostate cancer. Prostate. 2007 Jun 15;67(9):976-88) Außerdem bewirkt es eine vermehrte Ausschüttung bestimmter Interleukine (IL-15;IL-8).

Durch die erfindungsgemäße hohe lokale Konzentration von Lithiumionen in der gestenteten Läsion wird der Zellzyklus in der S-Phase gestoppt, wodurch eine Restenose vermindert wird, da vor allem sich schnell teilende Zellen betroffen sind. Ein Vorteil der Verwendung vom Lithiumsalzen liegt in der einfachen Verarbeitbarkeit dieser Verbindungen gegenüber den meisten organischen Wirkstoffen, die zu selben Zwecken Einsatz finden. Weiterhin betragen die Kosten derartiger Lithiumverbindungen nur einen Bruchteil organischer cytotoxischer Wirkstoffe, wie zum Beispiel von Sirolimus oder Paclitaxel.

Die Dosierung des Lithiumsalzes ist abhängig von vielerlei Faktoren, wie dem Implantationsort, der Art der Läsion, der Geometrie und der verwendeten Materialien des Implantats und dem allgemeinen Zustand des Patienten selbst. Im Gefäß sollte eine lokale Lithiumionen-Konzentration von 5 bis 30 mmol/l, mehr bevorzugt von 15 bis 25 mmol/l erreicht werden. Um dies zu erreichen wird der Stent mit 5 bis 50 µg pro mm Stentlänge eines Lithiumsalz beschichtet oder ein degradierbarer Stentgrundkörper derart hergestellt, dass er 1 bis 15 Gew.% Lithiumionen enthält, was einer Gesamtlithiummenge von 5 bis 200 µg pro mm Stentlänge entspricht.

Der Grundkörper weist eine Beschichtung auf, die aus einem Lithiumsalz besteht oder ein Lithiumsalz enthält. Eine Beschichtung im Sinne der Erfindung ist eine zumindest abschnittsweise Auftragung der Komponenten der Beschichtung auf den Grundkörper des Stents. Vorzugsweise wird die gesamte Oberfläche des Grundkörpers des Stents von der Beschichtung bedeckt. Eine Schichtdicke liegt vorzugsweise im Bereich von 1 nm bis 100 µm, besonders bevorzugt 300 nm bis 15 µm. Die Beschichtung besteht aus einem Lithiumsalz oder sie enthält ein solches Lithiumsalz. Der Gewichtsanteil des Lithiumsalzes an den die Beschichtung bildenden Komponenten der Beschichtung beträgt vorzugsweise mindestens 10%, besonders bevorzugt mindestens 30%. Die Beschichtung kann direkt auf die Implantatoberfläche aufgetragen werden. Die Verarbeitung kann nach Standardverfahren für die Beschichtung erfolgen. Es können einschichtige, aber auch mehrschichtige Systeme (zum Beispiel so genannte base coat -, drug coat - oder top coat - Schichten) erstellt werden.

Neben dem erfindungswesentlichen Lithiumsalz kann die Beschichtung weitere Bestandteile enthalten, insbesondere eine polymere Trägermatrix, in die das Lithiumsalz in fein dispergierter Form eingebettet ist. Mit anderen Worten, der Stent weist eine Beschichtung auf, die aus einer polymeren Trägermatrix mit eingebettetem Lithiumsalz besteht oder diese Komponenten enthält. Die Trägermatrix kann insbesondere weitere pharmazeutische Wirkstoffe, Röntgenmarker oder Magnetresonanzmarker enthalten.

Der Grundkörper des Stent besteht aus einem biokorrodierbaren Implantatwerkstoff und dieser Grundkörper enthält das Lithiumsalz. Bei bestimmungsgemäßer Verwendung des Stents wird der Grundkörper aus dem biokorrodierbaren Implantatwerkstoff allmählich abgebaut. Dabei wird das Lithiumsalz freigesetzt und kann die gewünschte pharmakologische Wirkung im Bereich der Läsion entfalten. Der biokorrodierbare Implantatwerkstoff wird in der Regel ein metallischer oder polymerer Implantatwerkstoff sein.

Biokompatible Metalle und Metalllegierungen für Permanentimplantate umfassen beispielsweise rostfreie Stähle (z. B. 316L), Kobaltbasislegierungen (z. B. CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder Ti-A16Nb7) und Goldlegierungen. Im Bereich biokorrodierbarer Stents ist der Einsatz von Magnesium oder Reineisen sowie biokorrodierbaren Basislegierungen der Elemente Magnesium, Aluminium, Zink, Eisen und Wolfram bevorzugt. Besonders bevorzugt ist der Einsatz einer biokorrodierbaren Magnesiumlegierung.

Unter einer biokorrodierbare Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die Magnesiumlegierung ist so in ihrer Zusammensetzung zu wählen, dass sie biokorrodierbar ist. Als Prüfmedium zur Testung des Korrosionsverhaltens von Legierungen dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe des zu untersuchenden Werkstoffs wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Besteht der Grundkörper des Stents aus einer biokorrodierbaren Magnesiumslegierung, so kann das Lithiumsalz sowohl Bestandteil einer Beschichtung des Grundkörpers sein als auch als Additiv dem Implantatwerkstoff zugesetzt werden. Es wird ein basisches Lithiumsalz eingesetzt, welches insbesondere in polaren organischen Lösungsmitteln löslich ist, um eine Mischung mit einer polymeren Trägermatrix zu ermöglichen. Insbesondere ist das Lithiumsalz ausgewählt aus der Gruppe umfassend Lithiumcabonat, Lithiumhydrogencarbonat, Lithiumhydroxid, Lithiumnitrid und einem Lithiumsalz einer Carbonsäureverbindung, zum Beispiel Lithiumstearat. Der besondere Vorteil basischer Lithiumsalze in Kombination mit biokorrodierbaren Magnesiumlegierungen besteht darin, dass die Korrosion des Stentimplantats in basischer Umgebung gehemmt ist. Ist der Grundkörper aus der biokorrodierbaren Magnesiumlegierung mit einer Beschichtung versehen, die aus einer polymeren Trägermatrix mit eingebettetem Lithiumsalz besteht oder diese Komponenten enthält, wobei die Trägermatrix ein biokorrodierbares Polymer ist, dessen Abbau zu einem sauren Abbauprodukt führt, so kann durch die Gegenwart basischer Lithiumsalze eine Beschleunigung des Abbaus des Grundkörpers verhindert werden. Weiterhin kann der Gefahr von Abstoßungsreaktionen durch die Gegenwart saurer Abbauprodukte des Polymers entgegengewirkt werden. Die genannten negativen Effekte können kompensiert oder bestenfalls sogar umgekehrt werden, wenn entsprechende Mengen an alkalischen Lithiumsalzen freigesetzt werden.

Besteht der Grundkörper des Stents aus einem biokorrodierbaren Polymer, so kann das Lithiumsalz sowohl Bestandteil einer Beschichtung des Grundkörpers sein als auch als Additiv dem Implantatwerkstoff zugesetzt werden. Es wird ein basisches Lithiumsalz eingesetzt. Beispiele derartiger Lithiumsalze umfassen Carbonsäuren, die insbesondere auch Substituenten wie beispielsweise Hydroxyl- oder Aminogruppen tragen können oder Mischsalze mit anderen Kationen darstellen. Beispiele derartiger Lithiumcarboxylate sind Lithiumformiat, Lithiumacetat, Lithiumlactat, Lithiumglycinat und Lithium-bis(oxalato)borat. Lithiumacetat ist besonders bevorzugt. Aber auch andere basische Salze wie Lithiumcarbonat, Lithiumhydrogencarbonat, Lithiumhydroxid, Lithiumnitrid können eingesetzt werden. Der Einsatz basischer Lithiumsalze ist besonders bevorzugt, wenn das biokorrodierbare Polymer ein Polymer ist, dessen Abbau zu einem sauren Abbauprodukt führt; insbesondere ist das biokorrodierbare Polymer ein Polyester, Polyesteramid oder Polyanhydrid. Durch den Zusatz basischer Lithiumsalze kann der Gefahr von Abstoßungsreaktionen durch die Gegenwart saurer Abbauprodukte des Polymers entgegengewirkt werden.

## Patentansprüche

1. Stent mit einem Grundkörper aus einem Implantatwerkstoff, wobei der Implantatwerkstoff biokorrodierbar ist und der Grundköper ein Lithiumsalz enthält
**dadurch gekennzeichnet, dass**
der Grundkörper eine Beschichtung aufweist, die aus einem Lithiumsalz besteht oder ein Lithiumsalz enthält, und das Lithiumsalz basisch ist.

2. Stent nach Anspruch 1, bei dem das Lithiumsalz ausgewählt ist aus der Gruppe umfassend Lithiumcarbonat, Lithiumhydrogencarbonat, Lithiumhydroxid, Lithiumnitrid und einem Lithiumsalz einer Carbonsäureverbindung.

3. Stent nach einem der vorhergehenden Ansprüche, bei dem der Implantatwerkstoff eine biokorrodierbare Magnesiumlegierung ist.

4. Stent nach einem der Ansprüche 1 bis 2, bei dem der Implantatwerkstoff ein biokorrodierbares Polymer ist.

5. Stent nach Anspruch 4, bei dem das biokorrodierbare Polymer ein Polymer ist, dessen Abbau zu einem sauren Abbauprodukt führt.

6. Stent nach Anspruch 5, bei dem das biokorrodierbare Polymer ein Polyester, ein Polyamid oder ein Polyanhydrid ist.

7. Stent nach einem der vorhergehenden Ansprüche, bei dem der Stent eine Beschichtung aufweist, die aus einer polymeren Trägermatrix mit eingebettetem Lithiumsalz besteht oder diese Komponenten enthält.

8. Stent nach Anspruch 7, bei dem die Trägermatrix ein biokorrodierbares Polymer ist, dessen Abbau zu einem sauren Abbauprodukt führt.

9. Stent nach Anspruch 8, bei dem das biokorrodierbare Polymer ein Polyester, ein Polyamid oder ein Polyanhydrid ist.

10. Verwendung eines Lithiumsalzes als Beschichtungsmaterial für einen Stent.

## Claims

1. A stent, having a main body formed of an implant material, wherein the implant material is biocorrodible and the main body contains a lithium salt, **characterised in that** the main body has a coating, which consists of a lithium salt or contains a lithium salt, and the lithium salt is alkaline.

2. The stent according to Claim 1, wherein the lithium salt is selected from the group comprising lithium carbonate, lithium hydrogen carbonate, lithium hydroxide, lithium nitride and a lithium salt of a carboxylic acid compound.

3. The stent according to one of the preceding claims, wherein the implant material is a biocorrodible magnesium alloy.

4. The stent according to one of Claims 1 to 2, wherein the implant material is a biocorrodible polymer.

5. The stent according to Claim 4, wherein the biocorrodible polymer is a polymer of which the degradation leads to an acid degradation product.

6. The stent according to Claim 5, wherein the biocorrodible polymer is a polyester, a polyamide or a polyanhydride.

7. The stent according to one of the preceding claims, wherein the stent has a coating, which consists of a polymer carrier matrix with embedded lithium salt or contains these components.

8. The stent according to Claim 7, wherein the carrier matrix is a biocorrodible polymer of which the degradation leads to an acid degradation product.

9. The stent according to Claim 8, wherein the biocorrodible polymer is a polyester, a polyamide or a polyanhydride.

10. Use of a lithium salt as a coating material for a stent.

## Revendications

1. Stent possédant un corps principal formé à partir d'un matériau d'implant, dans lequel le matériau d'implant est biocorrosif et le corps principal contient du sel de lithium, **caractérisé en ce que** le corps principal possède un revêtement constitué d'un sel de lithium ou contenant un sel de lithium, et le sel de lithium est de l'alcalin.

2. Stent selon la revendication 1, dans lequel le sel de lithium est sélectionné parmi le groupe comprenant du carbonate de lithium, du carbonate acide de lithium, de l'hydroxyde de lithium, du nitrure de lithium et un sel de lithium d'un composant d'acide carboxylique.

3. Stend selon l'une des revendications précédentes, dans lequel le matériau d'implant est un alliage de magnésium biocorrosif.

4. Stent selon l'une des revendications 1 à 2, dans lequel le matériau d'implant est un polymère biocorrosif.

5. Stent selon la revendication 4, dans lequel le polymère biocorrosif est un polymère dont la dégradation entraîne un produit de dégradation acide.

6. Stent selon la revendication 5, dans lequel le polymère biocorrosif est un polyester, un polyamide ou un polyanhydride.

7. Stent selon l'une des revendications précédentes, dans lequel le stent possède un revêtement constitué d'une matrice de support de polymère, dans laquelle est inclus du sel de lithium, ou contenant ces composants.

8. Stent selon la revendication 7, dans lequel la matrice de support est un polymère biocorrosif, dont la dégradation entraîne un produit de dégradation acide.

9. Stent selon la revendication 8, dans lequel le polymère biocorrosif est un polyester, un polyamide ou un polyanhydride.

10. Utilisation d'un sel de lithium comme matériau de revêtement pour un stent.
